# EUROPÄISCHE PATENTSCHRIFT

(11) **EP 3 003 176 B1**
(45) Veröffentlichungstag und Bekanntmachung des Hinweises auf die Patenterteilung: **07.02.2018**
(21) Anmeldenummer: 14734747.0
(22) Anmeldetag: 03.06.2014
(51) Int. Cl.: A61B 17/28, A61B 17/3201, A61B 17/88, B23D 29/02, B26B 17/02, A61B 90/00

(54) **ZANGE**
FORCEPS
PINCE

(30) Priorität: 04.06.2013 DE 102013105751
(43) Veröffentlichungstag der Anmeldung: 13.04.2016
(73) Patentinhaber: Karl Klappenecker GmbH & Co. KG, 78532 Tuttlingen-Nendingen (DE)
(72) Erfinder: SPITZNAGEL, Bernhard, 78606 Seitingen-Oberflacht (DE)
(74) Vertreter: Patentanwälte und Rechtsanwalt Weiß, Arat & Partner mbB
(86) Internationale Anmeldenummer: PCT/EP2014/061455
(87) Internationale Veröffentlichungsnummer: WO 2014/195295

(56) Entgegenhaltungen:
- DE-A1-102011 001 013
- DE-U1- 9 105 152
- DE-U1- 20 018 390
- FR-A1- 2 389 459
- GB-A- 139 528
- US-A- 103 152

## Beschreibung

### Technisches Gebiet

Die Erfindung betrifft eine Zange, insbesondere eine chirurgische Zange, nach dem Oberbegriff des Anspruchs 1.

### Stand der Technik

Aus dem Stand der Technik sind verschiedene chirurgische Zangen zum Schneiden von Implantaten und/oder medizinischen Drähten bekannt. Da zum Schneiden von Implantaten und/oder medizinischen Drähten oft hohe Scherkräfte erzeugt werden müssen, weisen diese Zangen meist mehrfache Übersetzungen, z.B. in Form von Kniehebelantrieben, auf. Weiterhin sind diese meist ergonomisch geformt, um vom Operateur mit einer Hand bedienbar zu sein.

In der EP 0 321 884 B1 ist eine Zange zum Schneiden von Draht dargestellt, die mit mehreren über Bolzen verbundenen Kniehebelantrieben ausgestattet ist. Diese sollen ein Schneiden auch von stärkeren Drähten mit einer Hand ermöglichen. Nachteilig ist jedoch, dass die Schneiden im geöffneten Zustand eine starke Spreizung zueinander aufweisen und nicht parallel zueinander ausgerichtet sind, weshalb die Möglichkeit besteht, dass der Draht beim Zusammenpressen der Zange aus den Scheiden heraus gleiten kann.

Eine weitere solche Zange mit mehreren Gelenken, die Kniehebelantriebe bilden, ist in der DE 20 207 785 U1 dargestellt. Diese weist in den Schneiden Ausnehmungen auf, die dazu gedacht sind, einen Draht aufzunehmen und ein Verrutschen zu verhindern. Jedoch weisen auch hier die Schneiden im geöffneten Zustand eine starke Spreizung zueinander auf.

Allgemein gilt im Bereich chirurgischer Instrumente, dass in jüngster Zeit die Reinigung von den komplexen Vorrichtungen ein wachsendes Problem darstellt. Während die Vorrichtungen technisch immer ausgefeilter werden, bedarf es zu ihrer Reinigung eines immer grösseren Aufwands. Dies gilt auch für die zuvor dargestellten Zangen aus der EP 321 884 B1 und DE 20 207 785 U1.

Insbesondere stellen hier die Teilbereiche der Zangen, welche mittels eines Reinigungsprozesses, insbesondere unter Verwendung von Reinigungsmittel, nicht oder nur bedingt in einen sterilen Zustand versetzt werden können, ein immenses Risiko dar.

Aus der DE 10 2011 001 013 A1 ist eine Zange zum Schneiden von Werkstücken, insbesondere aus Metall, mit zumindest einem Zangenschenkel und einem Druckelement bekannt, welche über einen Drehpunkt miteinander verbunden sind. Dabei soll dem Zangenschenkel ein Schneidelement mit einem Schneidmaul zugeordnet sein, wobei ein Grund des Zangenmauls und ein Grund des Schneidmauls nahe einem Drehpunkt angeordnet sind, um welchen das Schneidelement in dem Zangenschenkel dreht. Beim Bewegen des Druckelementes zum Zargenschenkel hin nimmt ein Druckstück das Schneidelement mit, so dass dieses, geführt durch einen Führungszapfen, in einer Führungsrinne und in einem Rollenkäfig um den Drehpunkt dreht.

Aus der FR 2 389 459 A1 ist eine weitere Zange bekannt, bei der an einem Zargenschenkel ein Ratschenrad um eine Drehachse drehbar angeordnet ist, wobei die Drehung durch den anderen Zangenschenkel bewirkt wird. Das Ratschenrad besitzt einen Kulissenstein, der mit seiner Oberfläche ein Schneidelement abläuft, an dem ein Maulteil vorgesehen ist.
Eine Zange der o.g. Art ist aus der GB 139 528 A bekannt, wobei einem Griffteil ein Druckhebel zugeordnet ist, der gegenüber dem Griffteil schwenkbar gelagert ist. An dem Griffteil ist ein Maulteil vorgesehen. Ferner ist zwischen dem Griffteil und dem Druckhebel ein Hebelarm angeordnet, welcher ein zweites Maulteil aufweist und von dem Druckhebel mit Druck beaufschlagbar ist. Der Hebelarm ist gegenüber dem Griffteil schwenkbar gelagert.

Eine chirurgische Zange der im Oberbegriff von Anspruch 1 genannten Art ist aus der DE 200 18 390 U1 bekannt.

### Aufgabe der Erfindung

Die Aufgabe der vorliegenden Erfindung ist es, die Nachteile des Standes der Technik zu überwinden. Insbesondere soll eine Zange bereitgestellt werden, die eine einfach erreichte, aber hohe Übersetzung ermöglicht. Weiterhin soll eine Zange bereitgestellt werden, bei der die Übersetzung ohne feste Verbindungen ausgebildet sein soll und die für verschiedene Zwecke genutzt werden kann.
Zudem sollen in Maulteilen eingebrachte Werkzeugelemente im geöffneten Zustand zumindest quasi-parallel zueinander ausgerichtet sein. Auch soll eine Zange bereitgestellt werden, bei der ohne großen Aufwand die Werkzeugelemente ausgetauscht werden können.

Zudem soll eine Zange bereitgestellt werden, die ohne grossen Aufwand bei einem Reinigungsprozess derart gereinigt werden kann, dass sie steril ist und weiterhin einfach in Einzelteile zerlegbar und ebenso einfach zusammensetzbar ist.

### Lösung der Aufgabe

Zur Lösung der Aufgabe führen die Merkmale nach dem Anspruch 1.

Zum Erreichen einer hohen Übersetzung trägt bei, dass zwischen dem Druckhebel und dem Hebelarm der Wälzkörper angeordnet ist, Dieser Wälzkörper ist dabei so ausgeführt, dass er es ermöglicht, eine Kraft vom Druckhebel auf den Hebelarm zu übertragen. Um dies zu erreichen, wird der Wälzkörper in einer Kulisse geführt. Zusätzlich weist der Hebelarm eine vorzugsweise konkave Aufnahme für den Wälzkörper auf. Dadurch entsteht eine Art Kniehebel, der zur hohen Übersetzung der Zange beiträgt. Der Wälzkörper und der dadurch entstehende Kniehebel sind in einem typischen Ausführungsbeispiel in der dem Maulteil zugewandten Seite des Gehäuses des Griffteils angeordnet.

Bei der Lagerung zwischen Griffteil und Druckhebel und der Lagerung zwischen Hebelarm und Griffteil handelt es sich um eine freie Lagerung. Diese Lagerungen befinden sich in einem Bereich des Griffteils, der als Teil eines Gehäuses ausgebildet ist. In einem typischen Ausführungsbeispiel sind die freien Lagerungen gelenkartig in Form eines Drehgelenks oder Drehschubgelenks ausgebildet.

Bei beiden freien Lagerungen handelt es sich um Wälzflächen, die ein Schwenken des Druckhebels und des Hebelarms zulassen. Vorzugsweise drehen der Hebelarm und/oder der Druckhebel dabei um einen Dreh-/Schwenkpunkt. Weiterhin besteht auch die Möglichkeit, das der Dreh-/Schwenkpunkt einen nicht punktuellen Bereich darstellt, in dem der Hebelarm und/oder der Druckhebel bei einer Betätigung der Zange innerhalb des Gehäuses schwenken können.

Um eine möglichst hohe Übersetzung zu erreichen, vorzugsweise mindestens eine Übersetzung von 1 zu 15, befinden sich die Dreh-/Schwenkpunkte von dem Druckhebel und dem Hebelarm in möglichst grossem Abstand zueinander.

Der Dreh-/Schwenkpunkt des Druckhebels befindet sich dabei auf einer dem Maulteil zugewandten Seite des Druckhebels. Dieses Ende des Druckhebels wird in einem typischen Ausführungsbeispiel in eine konkave Aufnahme, die sich auf der dem Maulteil zugewandten Seite des Gehäuses im Griffteil befindet, eingesetzt. Die konkave Aufnahme stellt weiterhin die Wälzfläche dar, die als Lager des Druckhebels, anstelle eines konventionellen Gelenks in Form eines Bolzen oder Niets, dient.

Der Dreh-/Schwenkpunkt des Hebelarms befindet sich auf einer dem Maulteil abgewandten Seite des Hebelarms. Das dem Maulteil abgewandte Ende des Hebelarms wird in einem typischen Ausführungsbeispiel in eine konkave Aufnahme, die sich auf der dem Maulteil abgewandten Seite des Gehäuses im Griffteil befindet, eingesetzt. Die konkave Aufnahme stellt auch hier die Wälzfläche dar, die als Lager des Hebelarms, anstelle eines konventionellen Gelenks in Form eines Bolzen oder Niets, dient.

Durch die grösstmögliche Entfernung der Lagerung und somit des Dreh-/Schwenkpunktes des Hebelarms vom Maulteil wird neben der hohen Übersetzung erreicht, dass die Maulteile auch im geöffneten Zustand quasi-parallel zueinander ausgerichtet sind.

Die Lagerungen zwischen Griffstück und Druckhebel, Griffstück und Hebelarm Druckhebel und Wälzkörper sowie Wälzkörper und Hebelarm weisen ähnliche bzw. dieselben Funktionen auf, die Gelenke in einer konventionellen Zange aufweisen. Daher handelt es sich in einem typischen Ausführungsbeispiel bei den konkaven Aufnahmen um eine Art Gelenkpfanne während es sich bei den Enden des Hebelarms und des Druckhebels um eine Art Gelenkkopf handelt. Ebenso handelt es sich in einem typischen Ausführungsbeispiel bei der dem Hebelarm zugewandten Seite des Wälzkörpers um eine Art Gelenkkopf.

Die Lagerung und somit der Dreh-/Schwenkpunkt zwischen dem Druckhebel und dem Griffstück sowie die Wälzflächen zwischen dem Wälzkörper und dem Druckhebel und zwischen dem Wälzkörper und dem Hebelarm befinden sich in einem typischen Ausführungsbeispiel auf der dem Maulteil zugewandten Seite des Gehäuses der Zange. Somit befindet sich in einem typischen Ausführungsbeispiel lediglich die Lagerung und somit der Dreh-/Schwenkpunkt zwischen Hebelarm und Griffstück auf der dem Maulteil abgewandten Seite des Gehäuses der Zange und somit weiter vom Maulteil entfernt.

Durch die Nutzung von Wälzflächen zur Lagerung und des Wälzkörpers zur Ausgestaltung eines Kniehebels kann die Reibung innerhalb der Zange auf ein geringes Mass reduziert werden. Dadurch kann eine an den Maulteilen wirkende, grosse Kraft erzeugt werden. Da zur Erzeugung der Kraft nur ein geringer Weg benötigt wird, kann die Zange mit einer Hand genutzt und bedient werden.

Weiterhin weisen in einem typischen Ausführungsbeispiel die Bauteile im Bereich der Lagerungen bzw. der Wälzflächen meist ungleiche Geometrien, insbesondere ungleiche Radien auf. Beispielsweise kann eine Dreh- und/oder Drehschubbewegung dadurch erreicht werden, dass der Radius der jeweiligen konkaven Aufnahmen in einem typischen Ausführungsbeispiel grösser ist als der Aussenradius des Endes des jeweiligen Bauteils, welches in der Aufnahme gelagert ist. Dadurch wird eine abwälzende Bewegung und somit eine geringe Reibung ermöglicht.

Die Kulisse zur Führung des Wälzkörpers ist im Griffteil und/oder einem Gehäusedeckel eingebracht. Der Gehäusedeckel ist zum Verschliessen des zumindest teilweise vom Griffteil dargestellten Gehäuses geeignet. Die Verbindung des Gehäusedeckels mit dem Griffteil erfolgt vorzugsweise durch Schrauben. Möglich ist aber auch das Nutzen einer Steckverbindung anstelle der Verschraubung.

Weiterhin weist das Griffteil innerhalb des Gehäusebereichs eine Einhängeausnehmung auf, die dazu geeignet ist, eine Feder aufzunehmen. Durch die Feder wird das Maul der Zange wieder geöffnet, sobald der Druckhebel nicht mehr in Richtung des Griffteils gedrückt wird.

Weiterhin weist das Griffteil am maulseitigen Ende eine Ausnehmung auf, die als ein geschlossener Kanal in Richtung einer Längsachse ausgebildet ist. Die Längsachse entspricht dabei etwa einer gedachten Verbindung zwischen dem maulseitigen Ende und dem maulabgewandten Ende des Griffteils. Die als geschlossener Kanal ausgebildete Ausnehmung ist in einem typischen Ausführungsbeispiel dazu geeignet, den Hebelarm aufzunehmen, der hierfür vom maulseitigen Ende des Griffstücks aus in den geschlossenen Kanal eingeschoben wird.

Durch die Ausbildung der Ausnehmung als geschlossener Kanal wird weiterhin erreicht, dass Griffteil und somit das Gehäuse in diesem Bereich eine höhere Stabilität aufweisen. Zudem können über die geschlossene Kontur der Ausnehmung einwirkende Kräfte besser in das Griffteil abgeleitet werden.

In einem typischen Ausführungsbeispiel sind die Maulteile der Zange zur Aufnahme von Werkzeugelementen geeignet. Diese werden vorzugsweise in dafür vorgesehenen Aufnahmen mit dem Maulteil verschraubt und somit im Griffteil und Hebelarm fixiert. Durch die Verschraubung ist es möglich, abgenutzte Werkzeugelemente auszuwechseln. Weiterhin besteht die Möglichkeit verschiedene Werkzeugelemente mit derselben Zange zu nutzen.

Vorzugsweise kommen Werkzeugelemente zum Schneiden von chirurgischen Implantaten, insbesondere Titan-Implantaten, zum Einsatz. Weiterhin sind auch Werkzeugelemente denkbar und vorliegend umfasst, die zum Schneiden von Draht oder zum Biegen, Prägen, Stanzen und/oder Zwicken von verschiedensten Werkstücken und/oder Implantaten, auch ausserhalb des medizinischen Bereichs, geeignet sind.

Die Werkzeugelemente zum Schneiden von chirurgischen Implantaten sind in Verbindung mit der hohen Übersetzung der Zange dazu geeignet auch Titan-Implantate mit grossen Stärken zu schneiden. Um dies zu erleichtern, weist zumindest eines der Werkzeugelemente zum Schneiden von chirurgischen Implantaten in einem typischen Ausführungsbeispiel Aufnahmestifte auf. Vorzugsweise ist die Form, Größe und/oder der Abstand der Aufnahmestifte auf die zu schneidenden chirurgischen Implantate angepasst. Durch die Aufnahmestifte kann das Implantat in der richtigen Position zwischen den Werkzeugelementen fixiert werden.

Um ein Verbiegen des Implantats an der vorgesehenen Schnittkante beim Schneiden zu verhindern, weisen die Werkzeugelemente in einem Ausführungsbeispiel Federandrücker auf. Diese bestehen vorzugsweise aus einem festen, aber trotzdem elastischen und verformbaren medizinischen Silikon und drücken das zu schneidende Implantat an das gegenüberliegende Werkzeugelement.

Durch die typische Ausführungsform der Werkzeugelemente zum Schneiden von chirurgischen Implantaten mit den Aufnahmestiften und den Federandrückern und die quasi-parallele Anordnung der Werkzeugelemente kann zudem eine sehr saubere Führung des Schneidguts gewährleistet werden. Weiterhin wird durch die quasi-parallele Ausrichtung der Werkzeugelemente im geöffneten Zustand gewährleistet, dass ein Schneidgut beim Schliessen der Zange nicht aus den Schneiden heraus gleitet.

In einem typischen Ausführungsbeispiel werden die Federandrücker in den Werkzeugelementen durch die Schrauben fixiert, die die Werkzeugelemente in den Maulteilen des Griffteils und des Hebelarms fixieren. Somit können auch die Federandrücker problemlos gewechselt werden.

Durch die Ausführungsform der Lager, als eine Art Wälzlager, liegen der Druckhebel und der Hebelarm im geöffneten Zustand der Zange nicht in den Lagerflächen an. Auch die Feder liegt im geöffneten Zustand locker im Gehäuse. Somit steht im geöffneten Zustand keines der einzelnen Bauteile der Zange unter Spannung. Da somit im weit geöffneten Zustand keine angepressten Flächen wie in einem konventionellen Gelenk vorhanden sind, kann die Zange dadurch ohne ein vorheriges Zerlegen steril gereinigt werden Da sämtliche Verbindungen der Zange lösbar oder gesteckt ausgestaltet sind, können die Einzelteile der Zange ohne weiteres auch einzeln gereinigt werden. Auch ein Austauschen einzelner Teile ist problemlos möglich. Dies hat den Vorteil, dass bei einem Defekt eines Einzelteils, wie etwa der Feder, im Gehäuse oder eines Federandrückers in den Werkzeugelementen, nicht die gesamte Zange neu beschafft werden muss. Weiterhin kann durch den einfachen Aufbau der Zange diese ohne weiteres vom medizinischen Personal zerlegt und wieder zusammengesetzt werden.

### Figurenbeschreibung

Weitere Vorteile, Merkmale und Einzelheiten der Erfindung ergeben sich aus der nachfolgenden Beschreibung bevorzugter Ausführungsbeispiele sowie anhand der Zeichnungen; diese zeigen in
- Figur 1: eine typische Ausführungsform einer erfindungsgemässen Zange mit geschlossenem Maul;
- Figur 2: das Ausführungsbeispiel nach Figur 1 ohne Gehäusedeckel mit geschlossenem Maul;
- Figur 3: das Ausführungsbeispiel nach Figur 2 mit geöffnetem Maul;
- Figur 4: eine Seitenansicht eines erfindungsgemässen Griffteils der Zange nach Figur 1;
- Figur 5: eine Ansicht von der proximalen Seite des Griffteils nach Figur 4;
- Figur 6: eine Draufsicht auf das Griffteil nach Figur 1;
- Figur 7: eine Seitenansicht eines erfindungsgemässen Druckhebels der Zange nach Figur 1;
- Figur 8: eine Seitenansicht eines erfindungsgemässen Druckhebels der Zange nach Figur 1;
- Figur 9: eine Ansicht von der proximalen Seite des Hebelarms nach Figur 8;
- Figur 10: eine Seitenansicht eines erfindungsgemässen Gehäusedeckels der Zange nach Figur 1;
- Figur 11: eine Ansicht von der proximalen Seite des Gehäusedeckels nach Figur 10;
- Figur 12: ein typisches Ausführungsbeispiel eines chirurgischen Implantats;
- Figur 13: eine Draufsicht auf ein Werkzeugelement für den Hebelarm einer erfindungsgemässen Zange;
- Figur 14: eine Vorderansicht des Werkzeugelements nach Figur 13;
- Figur 15: eine Seitenansicht des Werkzeugelements nach Figur 13;
- Figur 16: eine Draufsicht auf ein Werkzeugelement für das Griffteil einer erfindungsgemässen Zange;
- Figur 17: eine Vorderansicht des Werkzeugelements nach Figur 16;
- Figur 18: eine Seitenansicht des Werkzeugelements nach Figur 16;
- Figur 19: ein Schneidschema der Werkzeugelemente für ein Implantat.

### Ausführungsbeispiel

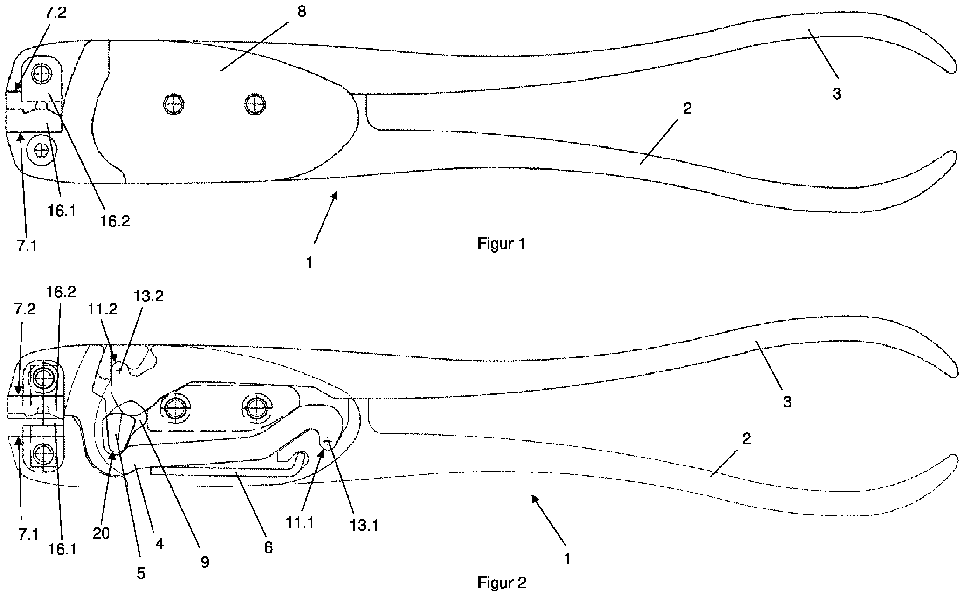

Figur 1 zeigt eine typische Ausführungsform einer erfindungsgemässen Zange 1 mit geschlossenem Maul. Eine erfindungsgemässe Zange umfasst dabei neben einem Griffteil 2 mit einem Maulteil 7.1 einen Druckhebel 3. Dieser Druckhebel 3 ist gegenüber dem Griffteil 2 schwenkbar gelagert.

In Figur 2 ist die Zange 1 nach Figur 1 ohne einen Gehäusedeckel 8 dargestellt. Dort ist ein Dreh-/Schwenkpunkt 13.2 dargestellt, um den der Druckhebel 3 in einem typischen Ausführungsbeispiel dreht bzw. schwenkt.

Dieser Dreh-/Schwenkpunkt befindet sich auf der den Maulteilen 7.1 und 7.2 zugewandten Seite des Druckhebels 3.

Weiterhin umfasst eine erfindungsgemässe Zange einen Hebelarm 4 mit einem zweiten Maulteil 7.2. Dieser ist in einem typischen Ausführungsbeispiel zwischen dem Griffteil 2 und dem Druckhebel 3 angeordnet und kann von dem Druckhebel 3 mit Druck beaufschlagt werden.

Dieser Hebelarm 4 ist in Figur 2 in einem in der Zange 1 eingesetzten Zustand dargestellt. Weiterhin ist dargestellt, dass auch der Hebelarm 4 einen Dreh-/Schwenkpunkt 13.1 gegenüber dem Griffteil 2 aufweist, um welchen der Hebelarm schwenkbar gegenüber dem Griffteil 2 gelagert ist. Dieser Dreh-/Schwenkpunkt 13.1 befindet sich auf der dem Maulteil 7.2 abgewandten Seite des Hebelarms 4, um eine möglichst hohe Übersetzung und zudem eine möglichst parallele Anordnung zu ermöglichen.

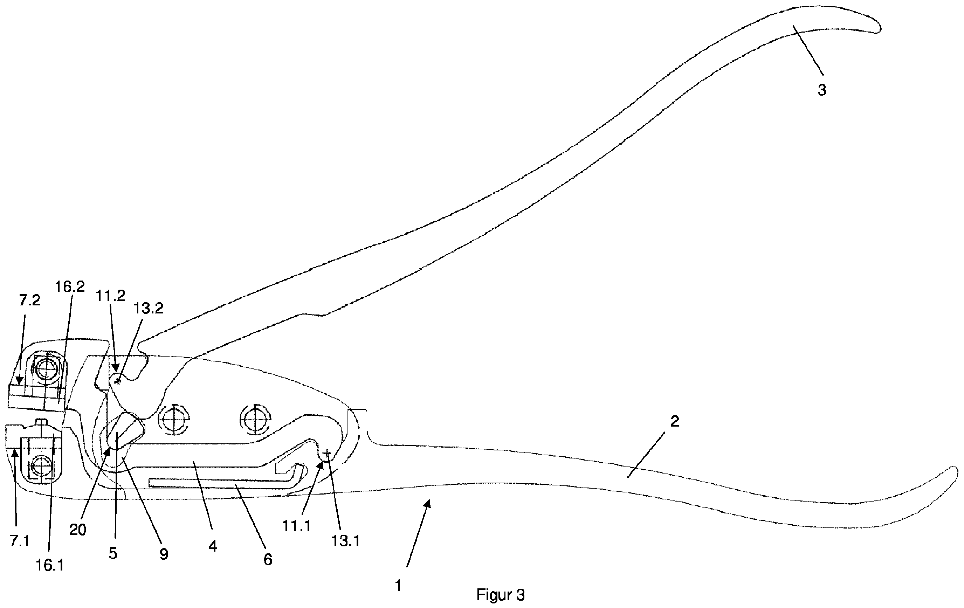

Weiterhin kann der Figur 2 und der Figur 3, die die erfindungsgemässe Zange 1 ohne Gehäusedeckel 8 im geöffneten Zustand zeigt, entnommen werden, dass es sich sowohl bei der Lagerung zwischen dem Druckhebel 3 und dem Griffteil 2 als auch bei der Lagerung zwischen dem Hebelarm 4 und dem Griffteil 2 um eine freie Lagerung handelt. Dies ermöglicht ein leichtes Zerlegen der Zange 1 durch einen Anwender.

Weiterhin ist in den Figuren 2 und 3 dargestellt, dass zwischen dem Druckhebel 3 und dem Hebelarm 4 ein Wälzkörper 5 angeordnet ist. Über den Wälzkörper 5 kann eine auf den Druckhebel 3 ausgeübte Druckkraft auf den Hebelarm 4 übertragen werden.

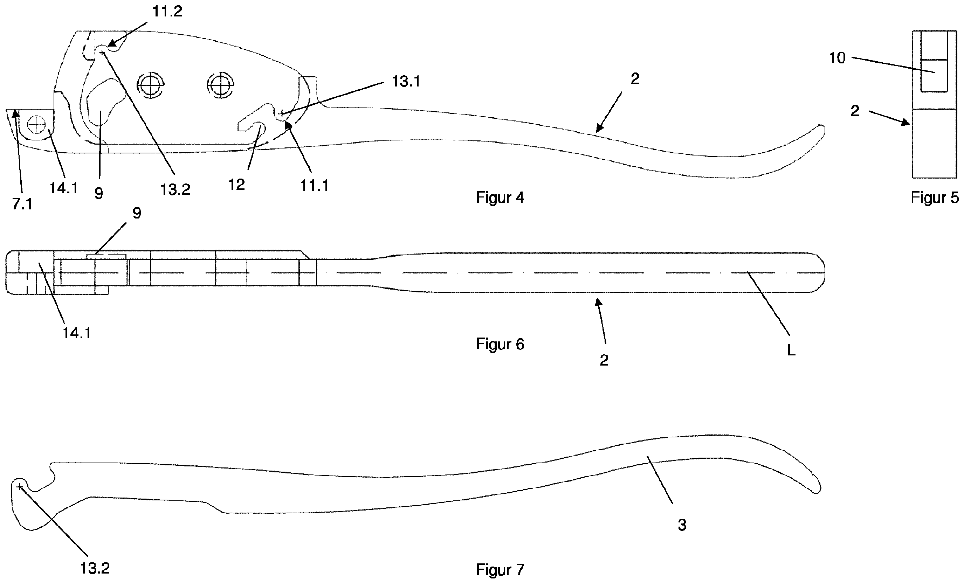

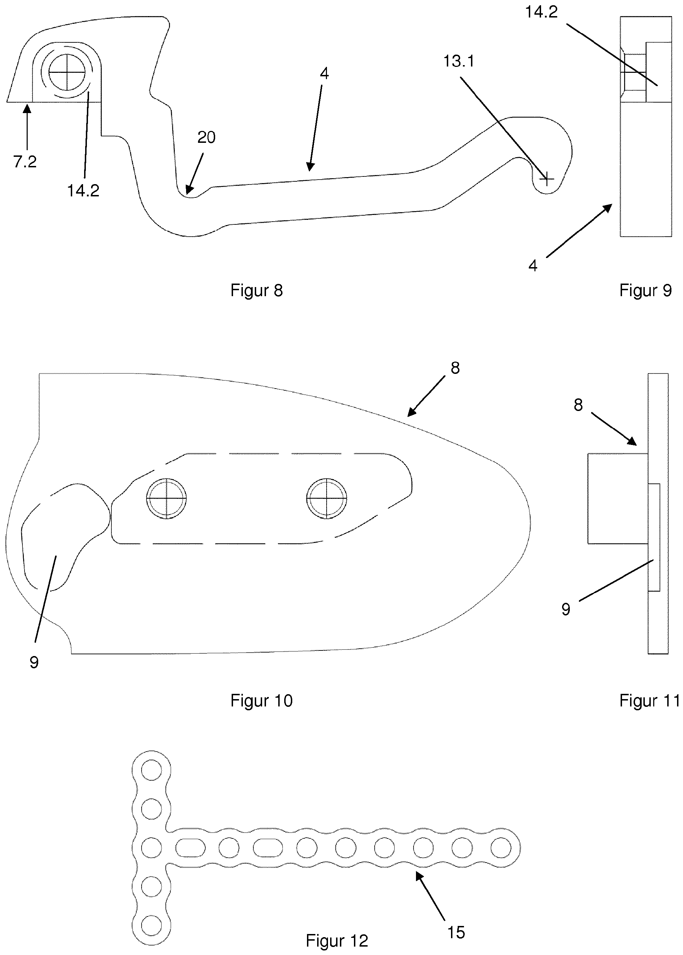

Um diese Kraft über den Wälzkörper 5 möglichst verlustfrei vom Druckhebel 3 auf den Hebelarm 4 zu übertragen, wird der Wälzkörper in einer Kulisse 9 geführt. Die Kulisse 9 ist hierfür in das Griffteil 2 und/oder den Gehäusedeckel 8 eingebracht. Das Griffteil 2 und die Lage der Kulisse im Griffteil 2 sind in den Figuren 4 und 6 näher dargestellt. Der Gehäusedeckel 8 und die Lage der Kulisse 9 im Gehäusedeckel sind in den Figuren 10 und 11 näher dargestellt. Weiterhin weist der Hebelarm 4 eine konkave Aufnahme 20 für den Wälzkörper 5 auf, die ebenfalls zu einer möglichst verlustfreien Kraftübertragung beiträgt. Um eine abwälzende Bewegung zu unterstützen, weist die konkave Aufnahme 20 für den Wälzkörper in einem typischen Ausführungsbeispiel einen Innenradius auf, der grösser als der Aussenradius des Wälzkörpers 5 im Bereich der Wälzfläche ist.

Aus den Figuren 4, 5 und 6 wird weiterhin ersichtlich, dass das Griffteil 2 zumindest teilweise ein Gehäuse aufweist, welches zur Aufnahme verschiedener Einzelteile der Zange 1 dient. Das Griffteil 2 weist auf der dem Maulteil 7.1 zugewandten Seite des Gehäuses eine konkave Aufnahme 11.2 auf. Die konkave Aufnahme 11.2 ist geeignet, das maulseitige Ende des Druckhebels 3 mit dem Dreh-/Schwenkpunkt 13.2 aufzunehmen. Die konkave Aufnahmen 11.2 weist in einem typischen Ausführungsbeispiel einen Innenradius auf, der grösser als der Aussenradius des Druckhebels 3 um den Dreh-/Schwenkpunkt 13.2 ist.

Auf der dem Maulteil 7.1 abgewandten Seite des Gehäuses weist das Griffstück 2 eine weitere konkave Aufnahme 11.1 auf. Die Aufnahme 11.1 ist geeignet, das dem Maulteil 7.2 gegenüberliegende Ende des Hebelarms 4 aufzunehmen, welches den Dreh-/Schwenkpunkt 13.1 aufweist. Die konkave Aufnahmen 11.1 weist in einem typischen Ausführungsbeispiel einen Innenradius auf, der grösser als der Aussenradius des Hebelarms 4 um den Dreh-/Schwenkpunkt 13.1 ist.

Weiterhin weist das Griffteil 2 auf der dem Maulteil 7.1 zugewandten Seite des Gehäuses eine Ausnehmung auf, die geeignet ist, den Hebelarm 4 aufzunehmen. Die Ausnehmung zur Aufnahme des Hebelarms 3 ist in einem typischen Ausführungsbeispiel in Form eines geschlossenen Kanals 10 in Richtung einer Längsachse L des Griffteils 2 ausgebildet. Somit wird beim Zusammensetzten der Zange 1 der Hebelarm 4 von der maulseitigen Seite aus in den geschlossenen Kanal 10 eingeführt.

Weiterhin weist das Griffteil 2 eine Einhängeausnehmung 12 für eine Feder 6 auf, welche dazu geeignet ist, die Zange 1 aus einer Gebrauchslage mit geschlossenem Maul in eine Gebrauchslage mit geöffneten Maul zu bringen. Das Gehäuse der Zange 1, welches zumindest teilweise vom Griffteil 1 gebildet wird, wird, wie in Figur 1 dargestellt, mit dem Gehäusedeckel 8 verschlossen. Eine feste Verbindung des Griffteils mit dem Gehäusedeckel kann dabei über Schrauben und/oder eine Steckverbindung erfolgen. Vorzugsweise erfolgt die Verbindung über in den Figuren nicht näher dargestellte Schrauben.

Die Maulteile 7.1 und 7.2 der Zange 1 sind in einer erfindungsgemässen Ausführungsform der Zange auch im geöffneten Zustand quasi-parallel zueinander ausgerichtet. Weiterhin sind die Maulteile 7.1 und 7.2 dazu geeignet, Werkzeugelemente 16.1 und 16.2 aufzunehmen. In einem typischen Ausführungsbeispiel sind die Werkzeugelemente 16.1 und 16.2 auswechselbar. Vorzugsweise handelt es sich bei den Werkzeugelementen 16.1 und 16.2 um Einsätze, die zum Schneiden von chirurgischen Implantaten 15 geeignet sind. In Figur 12 ist eine Ausführungsform eines solchen Implantats 15 dargestellt. Durch die Anordnung der Dreh-/Schwenkpunkte 13.1 und 13.2 wird ein ausreichend hohes Übersetzungsverhältnis erreicht, um auch hochfeste Titan-Implantate zu schneiden.

In einer erfindungsgemässen Ausführungsform der Zange 1 sind die Werkzeugelemente 16.1 und 16.2 durch Verschraubungen in den Aufnahmen 14.1 und 14.2 fixiert, die in den Maulteilen 7.1 und 7.2 des Griffteils 2 und des Hebelarms 4 angeordnet sind. Um das chirurgische Implantat 15 sauber und effektiv zu durchtrennen, sind die Schneiden der Werkzeugelemente 16.1 und 16.2 in einem typischen Ausführungsbeispiel aufeinander und auf das chirurgische Implantat 15 angepasst.

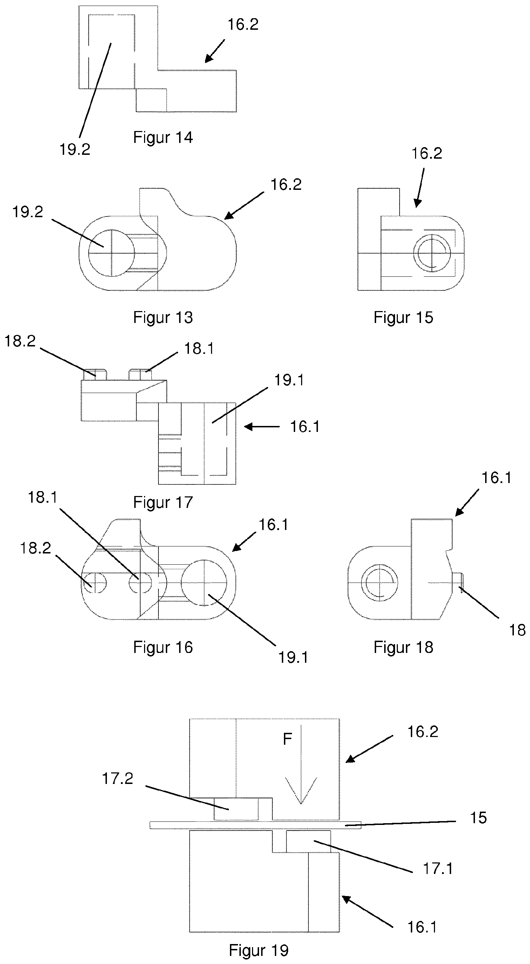

Weiterhin weist, wie in den Figuren 16 bis 18 dargestellt, zumindest eines der Werkzeugelemente 16.1 und/oder 16.2 Aufnahmestifte 18.1 und 18.2 auf, die ein Verrutschen des chirurgischen Implantats 15 verhindern und das chirurgische Implantat 15 somit fixieren. Weiterhin kann durch die Aufnahmestifte 18.1 und 18.2 gewährleistet werden, dass die Stegbreite an der Schnittkante des chirurgischen Implantats 15 eine ausreichende Stärke aufweist.

In einem typischen Ausführungsbeispiel weisen die Werkzeugelemente 16.1.und 16.2 zudem Ausnehmungen auf, die als Aufnahme 19.1 und 19.2 für Federandrücker 17.1 und 17.2 dienen. Durch die Federandrücker 17.1 und 17.2 wird ein Verbiegen des Implantats 15 beim Schneiden verhindert. Dargestellt ist dies in Figur 19, die das Schneidschema für ein typisches Ausführungsbeispiel darstellt.

Um ein vollständiges Schliessen des Mauls der Zange 1 zu ermöglichen, sind die Federandrücker 17.1 und 17.2 vorzugsweise aus einem festen, aber elastischen und somit verformbaren Silikon hergestellt. Die Federandrücker 17.1 und 17.2 werden in einem typischen Ausführungsbeispiel durch die Verschraubungen in den Werkzeugelementen 16.1 und 16.2 fixiert, die die Werkzeugelemente 16.1 und 16.2 in den Aufnahmen 14.1 und 14.2 der Maulteile 7.1 und 7.2 fixieren.

Im Folgenden wird die Funktionsweise der Zange 1 mit Werkzeugelementen 16.1 und 16.2 zum Schneiden von chirurgischen Implantaten 15 dargestellt. Die Werkzeugelemente 16.1 und 16.2 zum Schneiden von chirurgischen Implantaten sind in den Figuren 14 bis 18 dargestellt. Das dazugehörige Schneidschema ist in Figur 19 dargestellt:
Um ein Implantat 15 zu schneiden, wird dieses in das geöffnete Maul der Zange 1 eingelegt. Durch die Aufnahmestifte 18.1 und 18.2 wird eine möglichst ideale Positionierung des Implantats15 zwischen den Werkzeugelementen 16.1.und 16.2 im Maulteil der Zange 1 erreicht.

Durch das Ausüben einer Kraft auf den Druckhebel 3 schwenkt dieser um den Dreh-/Schwenkpunkt 13.2 und übt eine Kraft auf den Wälzkörper 5 aus. Dieser wird hierdurch in der Kulisse 9 in Richtung des Hebelarms 4 geführt. Dadurch beginnt der Hebelarm 4 um den Dreh-/Schwenkpunkt 13.1 zu schwenken. Dadurch bewegt sich das Maulteil 7.2 des Hebelarms 4 in Richtung des Maulteils 7.1 des Griffteils 2.

Durch die Federandrücker 17.1 und 17.2 und die Aufnahmestifte 18.1 und 18.2 wird das Implantat 15 zwischen den Werkzeugelementen 16.1 und 16.2 fixiert. Durch das Zusammendrücken wirkt nun eine Kraft F auf das Implantat 15 und dieses wird dadurch an der vorgesehenen Schnittkante durchtrennt.

**Bezugszeichenliste**

| | | | | | |
|---|---|---|---|---|---|
| 1 | Zange | 34 | | L | Längsachse |
| 2 | Griffteil | 35 | | F | Kraft |
| 3 | Druckhebel | 36 | | | |
| 4 | Hebelarm | 37 | | | |
| 5 | Wälzkörper | 38 | | | |
| 6 | Feder | 39 | | | |
| 7 | Maulteil | 40 | | | |
| 8 | Gehäusedeckel | 41 | | | |
| 9 | Kulisse | 42 | | | |
| 10 | geschlossener Kanal | 43 | | | |
| 11 | konkave Aufnahme | 44 | | | |
| 12 | Einhängeausnehmung | 45 | | | |
| 13 | Dreh-/Schwenkpunkt | 46 | | | |
| 14 | Aufnahme Werkzeugelement | 47 | | | |
| 15 | Implantat | 48 | | | |
| 16 | Werkzeugelement | 49 | | | |
| 17 | Federandrücker | 50 | | | |
| 18 | Aufnahmestift | 51 | | | |
| 19 | Aufnahme Federandrücker | 52 | | | |
| 20 | Aufnahme Wälzkörper | 53 | | | |
| 21 | | 54 | | | |
| 22 | | 55 | | | |
| 23 | | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Zange (1), insbesondere chirurgische Zange, mit zumindest einem Griffteil (2) und einem Druckhebel (3), der gegenüber dem Griffteil schwenkbar gelagert ist und mit einem Maul aus zwei Maulteilen (7.1, 7.2), wobei am Griffteil (2) eines der Maulteile (7.1) vorgesehen ist und wobei zwischen dem Griffteil (2) und dem Druckhebel (3) ein Hebelarm (4) angeordnet ist, welcher das zweite Maulteil (7.2) aufweist und von dem Druckhebel (3) mit Druck beaufschlagbar und der Hebelarm (4) gegenüber dem Griffteil (2) schwenkbar gelagert ist, wobei zwischen dem Druckhebel (3) und dem Hebelarm (4) ein Wälzkörper (5) angeordnet undeine auf den Druckhebel (3) ausgeübte Kraft über den Wälzkörper (5) auf den Hebelarm (4) übertragbar ist,
**dadurch gekennzeichnet,**
**dass** der Wälzkörper (5) in einer Kulisse (9) geführt ist, die Kulisse (9) zum Führen des Wälzkörpers (5) in dem Griffteil (2) und/oder einem Gehäusedeckel (8) eingebracht ist und der Hebelarm (4) eine konkave Aufnahme (20) für den Wälzkörper (5) mit einem Innenradius aufweist, der grösser als ein Aussenradius des Walzenkörpers (5) im Bereich der Wälzflächen ist, wobei. durch Ausüben einer Kraft auf den Druckhebel (3) dieser um einen Dreh-/Schwenkpunkt (13.2) schwenkt und eine Kraft auf den Wälzkörper (5) ausübt, der hierdurch in der Kulisse (9) in Richtung des Hebelarms (4) führbar ist, wodurch der Hebelarm (4) um einen Dreh-/Schwenkpunkt (13.1) zu schwenken beginnt und sich das Maulteil (7.2) des Hebelarms (4) in Richtung des Maulteils (7.1) des Griffteils (2) bewegt, und die Lagerung zwischen Griffteil (2) und Druckhebel (3) und zwischen dem Griffteil (2) und dem Hebelarm (4) eine freie Lagerung ist.

2. Zange (1) Anspruch 1, **dadurch gekennzeichnet, dass** sich ein Dreh-/Schwenkpunkt (13.1) des Hebelarms (4) auf der dem Maulteil (7.2) abgewandten Seite befindet.

3. Zange (1) nach einem der vorherigen Ansprüche, **dadurch gekennzeichnet, dass** sich ein Dreh-/Schwenkpunkt (13.2) des Druckhebels (3) auf der den Maulteilen (7.1, 7.2) zugewandten Seite des Druckhebels (3) befindet.

4. Zange (1) nach einem der vorherigen Ansprüche, **dadurch gekennzeichnet, dass** das Griffteil (2) zumindest teilweise ein Teil eines Gehäuses ist.

5. Zange (1) nach einem der vorherigen Ansprüche, **dadurch gekennzeichnet, dass** das Griffteil (2) an einem maulseitigen Ende eine konkave Aufnahme (11,2) für den Druckhebel (3) aufweist.

6. Zange (1) nach einem der vorherigen Ansprüche, **dadurch gekennzeichnet, dass** Griffteil (2) an einem maulabgewandten Ende eine konkave Aufnahme (11.1) für den Hebelarm (4) aufweist.

7. Zange (1) nach einem der vorherigen Ansprüche, **dadurch gekennzeichnet, dass** das Griffteil (2) am maulseitigen Ende eine Ausnehmung aufweist, welche geeignet ist, den Hebelarm (4) aufzunehmen.

8. Zange (1) nach Anspruch 7, **dadurch gekennzeichnet, dass** die Ausnehmung zur Aufnahme des Hebelarms (4) als geschlossener Kanal (10) in Richtung einer Längsachse (L) des Griffteils (2) ausgebildet ist.

9. Zange (1) nach einem der vorherigen Ansprüche, **dadurch gekennzeichnet, dass** das Griffteil (2) eine Einhängeausnehmung (12) aufweist, welche geeignet ist, eine Feder (6) aufzunehmen.

10. Zange (1) nach einem der vorherigen Ansprüche 1 bis 9, **dadurch gekennzeichnet, dass** der Gehäusedeckel (8) zum Verschliessen des Gehäuses geeignet ist.

11. Zange (1) nach einem der vorherigen Ansprüche, **dadurch gekennzeichnet, dass** die Maulteile (7.1, 7.2) quasi-parallel zueinander ausgerichtet sind.

12. Zange (1) nach einem der vorherigen Ansprüche, **dadurch gekennzeichnet, dass** die Maulteile (7.1, 7.2) zur Aufnahme von Werkzeugelementen (16.1, 16.2) geeignet sind, die auswechselbar sind.

13. Zange (1) nach Anspruch 12, **dadurch gekennzeichnet, dass** zumindest eines der werkzeugelemante (16.1, 16.2) zum Schneiden von Implantaten Aufnahmestifte (18.1, 18.2) zur Aufnahme und Fixierung des Implantats (15) aufweist.

14. Zange (1) nach Anspruch 12 oder 13, **dadurch gekennzeichnet, dass** die Werkzeugelemente (16.1. 16.2) zum Schneiden von Implantaten (15) Federandrücker (17.1, 17.2) aufweisen.

15. Zange (1) nach einem der Ansprüche 12 bis 14, **dadurch gekennzeichnet, dass** die Federandrücker (17.1. 17.2) in den Werkzeugelementen (16.1, 16.2) zum Schneiden von Implantaten (15) durch die Schrauben in den Werkzeugelementen (16.1, 16.2) fixiert sind, welche die Werkzeugelemente (16.1, 16.2) in dem Griffteil (2) und/oder Hebelarm (4) fixieren.

## Claims

1. Forceps (1), in particular surgical forceps, comprising at least one handle part (2) and a pressure lever (3) which is mounted pivotably in relation to the handle part, and comprising a jaw consisting of two jaw parts (7.1, 7.2), wherein one of the jaw parts (7.1) is provided on the handle part (2) and wherein a lever arm (4) which has the second jaw part (7.2) and is pressurizable by the pressure lever (3) is arranged between the handle part (2) and the pressure lever (3) and the lever arm (4) is mounted pivotably in relation to the handle part (2), wherein a rolling body (5) is arranged between the pressure lever (3) and the lever arm (4) and a force exerted on the pressure lever (3) is transmittable via the rolling body (5) to the lever arm (4),
**characterized**
**in that** the rolling body (5) is guided in a slotted guide (9), the slotted guide (9) for guiding the rolling body (5) is provided in the handle part (2) and/or in a housing cover (8) and the lever arm (4) has a concave receptacle (20) for the rolling body (5) with an internal radius which is larger than an external radius of the rolling body (5) in the region of the rolling surfaces, wherein, by exerting a force on the pressure lever (3), the latter pivots about a fulcrum/pivot point (13.2) and exerts a force on the rolling body (5) which is thereby guided in the slotted guide (9) in the direction of the lever arm (4), as a result of which the lever arm (4) begins to pivot about a fulcrum/pivot point (13.1) and the jaw part (7.2) of the lever arm (4) moves in the direction of the jaw part (7.1) of the handle part (2), and the mounting between handle part (2) and pressure lever (3) and between the handle part (2) and the lever arm (4) is a free mounting.

2. Forceps (1) claim 1, **characterized in that** a fulcrum/pivot point (13.1) of the lever arm (4) is located on the side facing away from the jaw part (7.2).

3. Forceps (1) according to one of the preceding claims, **characterized in that** a fulcrum/pivot point (13.2) of the pressure lever (3) is located on the side of the pressure lever (3) which faces the jaw parts (7.1, 7.2).

4. Forceps (1) according to one of the preceding claims, **characterized in that** the handle part (2) is at least partially a part of a housing.

5. Forceps (1) according to one of the preceding claims, **characterized in that** the handle part (2) has, at a jaw-side end, a concave receptacle (11.2) for the pressure lever (3).

6. Forceps (1) according to one of the preceding claims, **characterized in that** the handle part (2) has, at an end facing away from the jaw, a concave receptacle (11.1) for the lever arm (4).

7. Forceps (1) according to one of the preceding claims, **characterized in that** the handle part (2) has, at the jaw-side end, a recess which is suitable for receiving the lever arm (4).

8. Forceps (1) according to claim 7, **characterized in that** the recess for receiving the lever arm (4) is designed as a closed channel (10) in the direction of a longitudinal axis (L) of the handle part (2).

9. Forceps (1) according to one of the preceding claims, **characterized in that** the handle part (2) has a hook-in recess (12) which is suitable for receiving a spring (6).

10. Forceps (1) according to one of the preceding claims, **characterized in that** the housing cover (8) is suitable for closing the housing.

11. Forceps (1) according to one of the preceding claims, **characterized in that** the jaw parts (7.1, 7.2) are aligned virtually parallel to each other.

12. Forceps (1) according to one of the preceding claims, **characterized in that** the jaw parts (7.1, 7.2) are suitable for receiving tool elements (16.1, 16.2) which are exchangeable.

13. Forceps (1) according to claim 12, **characterized in that** at least one of the tool elements (16.1, 16.2) for cutting implants has receiving pins (18.1, 18.2) for receiving and securing the implant (15).

14. Forceps (1) according to claim 12 or 13, **characterized in that** the tool elements (16.1, 16.2) for cutting implants (15) have spring pressers (17.1, 17.2).

15. Forceps (1) according to one of claims 12 to 14, **characterized in that** the spring pressers (17.1, 17.2) are secured in the tool elements (16.1, 16.2) for cutting implants (15) by means of the screws in the tool elements (16.1, 16.2), which screws secure the tool elements (16.1, 16.2) in the handle part (2) and/or lever arm (4).

## Revendications

1. Pince (1), en particulier pince chirurgicale, avec au moins une partie de poignée (2) et un levier de pression (3) qui est monté de manière pivotante par rapport à la partie de poignée et avec une mâchoire composée de deux parties de mâchoire (7.1, 7.2), dans laquelle à la partie de poignée (2) est prévue l'une des parties de mâchoire (7,1) et dans laquelle entre la partie de poignée (2) et le levier de pression (3) est disposé un bras de levier (4) qui présente la deuxième partie de mâchoire (7.2) et peut être soumis à de la pression par le levier de pression (3) et le bras de levier (4) est monté de manière pivotante par rapport à la partie de poignée (2), dans lequel un élément de roulement (5) est disposé entre le levier de pression (3) et le bras de levier (4) et une force exercée sur le levier de pression (3) peut être transmise par l'intermédiaire de l'élément de roulement (5) au bras de levier (4),
**caractérisée par le fait**
**que** le corps de roulement (5) est guidé dans une coulisse (9), la coulisse (9) est placée pour le guidage de l'élément de roulement (5) dans la partie de poignée (2) et/ou dans un couvercle de boîtier (8) et le bras de levier (4) présente un réceptacle concave (20) pour l'élément de roulement (5) avec un rayon intérieur qui est supérieur à un rayon extérieur de l'élément de roulement (5) dans la zone des surfaces de roulement, où ce dernier pivote, en exerçant une force sur le levier de pression (3), autour d'un point de rotation/de pivotement (13.2) et exerce une force sur l'élément de roulement (5) qui peut de ce fait être guidé dans la coulisse (9) en direction du bras de levier (4), d'où le bras de levier (4) commence à pivoter autour d'un point de rotation/de pivotement (13.1) et la partie de mâchoire (7.2) du bras de levier (4) se déplace en direction de la partie de mâchoire (7.1) de la partie de poignée (2), et le montage entre la partie de poignée (2) et le levier de pression (3) et entre la partie de poignée (2) et le bras de levier (4) est un montage libre.

2. Pince (1) selon la revendication 1, **caractérisée par le fait qu'**un point de rotation/ de pivotement (13.1) du bras de levier (4) se trouve du côté opposé à la partie de mâchoire (7.2).

3. Pince (1) selon l'une des revendications précédentes, **caractérisée par le fait qu'**un point de rotation/de pivotement (13.2) du levier de pression (3) se trouve du côté orienté vers les parties de mâchoire (7.1, 7.2) du levier de pression (3).

4. Pince (1) selon l'une des revendications précédentes, **caractérisée par le fait que** la partie de poignée (2) est au moins en partie une partie d'un boîtier.

5. Pince (1) selon l'une des revendications précédentes, **caractérisée par le fait que** la partie de poignée (2) présente, à une extrémité du côté de la mâchoire, un réceptacle concave (11.2) pour le levier de pression (3).

6. Pince (1) selon l'une des revendications précédentes, **caractérisée par le fait que** la partie de poignée (2) présente, à une extrémité opposée à la mâchoire, un réceptacle concave (11.1) pour le bras de levier (4).

7. Pince (1) selon l'une des revendications précédentes, **caractérisée par le fait que** la partie de poignée (2) présente, à l'extrémité du côté de la mâchoire, un évidement qui convient pour recevoir le bras de levier (4).

8. Pince (1) selon la revendication 7, **caractérisée par le fait que** l'évidement destiné à recevoir le bras de levier (4) est réalisé sous forme d'un canal fermé (10) en direction d'un axe longitudinal (L) de la partie de poignée (2).

9. Pince (1) selon l'une des revendications précédentes, **caractérisée par le fait que** la partie de poignée (2) présente un évidement d'accrochage (12) qui convient pour recevoir un ressort (6).

10. Pince (1) selon l'une des revendications précédentes 1 à 9, **caractérisée par le fait que** le couvercle de boîtier (8) convient pour fermer le boîtier.

11. Pince (1) selon l'une des revendications précédentes, **caractérisée par le fait que** les parties de mâchoire (7.1, 7.2) sont orientées quasi parallèlement l'une à l'autre.

12. Pince (1) selon l'une des revendications précédentes, **caractérisée par le fait que** les parties de mâchoire (7.1, 7.2) conviennent pour recevoir des éléments d'outil (16.1, 16.2) qui sont interchangeables.

13. Pince (1) selon la revendication 12, **caractérisée par le fait qu'**au moins l'un des éléments d'outil (16.1, 16.2) pour couper des implants présente des broches de réception (18.1, 18.2) pour la réception et la fixation des implants (15).

14. Pince (1) selon la revendication 12 ou 13, **caractérisée par le fait que** les éléments d'outil (16.1, 16.2) pour couper des implants (15) présentent des poussoirs à ressort (17.1, 17.2).

15. Pince (1) selon l'une des revendications 12 à 14, **caractérisée par le fait que** les poussoirs à ressort (17.1, 17.2) dans les éléments d'outil (16.1, 16.2) pour couper des implants (15) sont fixés par les vis dans les éléments d'outil (16.1, 16.2) qui fixent les éléments d'outil (16.1, 16.2) dans la partie de poignée (2) et/ou le bras de levier (4).
